# EUROPEAN PATENT SPECIFICATION

(11) **EP 2 087 848 B1**
(45) Date of publication and mention of the grant of the patent: **27.12.2017**
(21) Application number: 09152267.2
(22) Date of filing: 06.02.2009
(51) Int. Cl.: A61B 18/14

(54) **End effector assembly for electrosurgical device and method for making the same**
Endeffektoranordnung für elektrochirurgische Vorrichtung und Herstellungsverfahren dafür
Ensemble effecteur d'extrémité pour dispositif électrochirurgical et son procédé de fabrication

(30) Priority: 06.02.2008 US 26499 P; 28.01.2009 US 361375
(43) Date of publication of application: 12.08.2009
(73) Proprietor: Covidien LP, Mansfield, MA 02048 (US)
(72) Inventor: Chojin, Edward M., Boulder, CO 80301 (US)
(74) Representative: Soames, Candida Jane

(56) References cited:
- EP-A- 1 535 581
- EP-A- 1 609 430
- JP-A- 61 115 672
- US-A- 2 031 138
- US-A- 5 202 545
- US-A1- 2007 106 297

## Description

### BACKGROUND

### Technical Field

The present disclosure relates to an electrosurgical forceps and more particularly, the present disclosure relates to electrosurgical forceps, which employ an end effector assembly configured for receiving a knife blade, or portion thereof, within a seal plate.

### Description of Related Art

As is known in the art, bipolar forceps typically employ end effector assemblies that include one or more jaw members. Generally, the jaw members include a structural support member, an insulative member, and a thin seal plate. The structural support member and the thin seal each may include a slot adapted to receive a knife blade, or portion thereof.

The structural support and the thin seal plate of the jaw members may be over-molded and/or stamped together, wherein the slot of the seal plate is configured to align with a slot or groove on the structural support member such that when the jaw members are in a closed configuration, the knife blade can move longitudinally therein. Because the knife blade, the slot in the structural support member, and slot in the seal plate need to be in almost perfect alignment with each other, machining tolerances during the manufacture process must be kept at a minimum. Thus, manufacturing conventional end-effector assemblies is generally time-consuming and requires a multi-step process, which in turn may lead to increased cost to the manufacturer. US2007/0106297 discloses an endoscopic forceps. An electrically conductive sealing plate and an outer housing form a longitudinally oriented knife slot when assembled.

### SUMMARY OF THE DISCLOSURE

An end effector assembly for use with a forceps is provided. The end effector assembly including a pair of opposing jaw members operatively connected to each other about a pivot. Each jaw member includes a structural support member having a seal plate secure thereto. Each of the structural supports includes one or more apertures extending therein configured as further defined in claim 1. In embodiments, the aperture may extend through the entire thickness of the seal plate. Alternatively, the aperture does not extend through the entire thickness of the seal plate. Each of the seal plates defines a slot having a depth and configured to translate a knife blade therethrough. The combined depth of the slot is greater than the height of the knife blade.

In an embodiment, the seal plates are secured to their respective structural support members via at least one of welding, soldering and brazing. The type of weld may be selected from the group consisting of resistance welding, ultrasonic welding, gas welding, energy beam welding, explosion welding, and solid state welding.

A method for manufacturing an end effector assembly for use with a bipolar forceps is provided. The method including the steps of: a) providing a structural support, the structural support including at least one aperture associated with a top surface thereof; b) providing a seal plate, the seal plate defining a slot configured to translate a knife blade therethrough; and c) utilizing the at least one aperture to gain access to couple the seal plates to the structural support.

### BRIEF DESCRIPTION OF THE DRAWING

FIG. 1 is a perspective view of a bipolar forceps in accordance with an embodiment of the present disclosure;

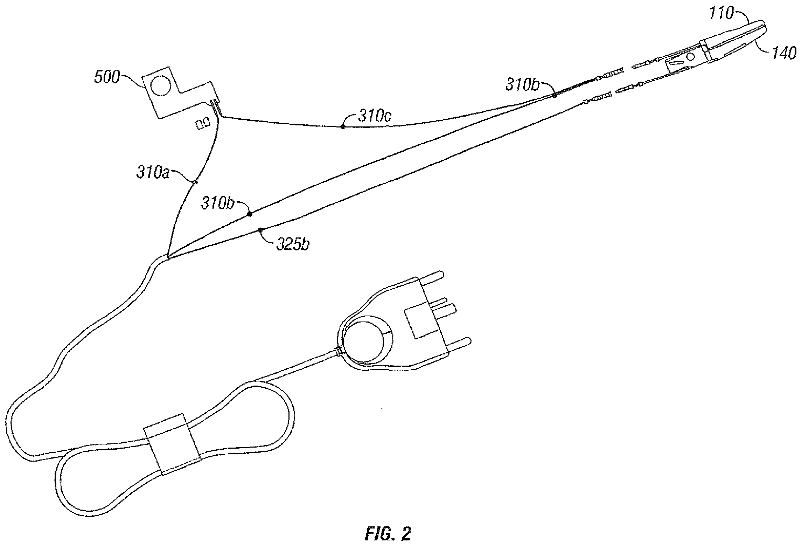

FIG. 2 illustrates an electrical wiring diagram for the bipolar forceps depicted in FIG. 1 in accordance with the present disclosure;
FIG. 3A is an exploded view of the area defined by reference numeral 3 in FIG. 1 illustrating a structural support member and a seal plate in accordance with an embodiment of the present disclosure;
FIG. 3B is an exploded view of the area defined by reference numeral 3 in FIG. 1 illustrating a structural support member and a seal plate in accordance with an alternate embodiment of the present disclosure;
FIGS. 4A and 4B are side and cross sectional views, respectively, illustrating a knife blade within the seal plate depicted in FIG. 3B;
FIGS. 5A and 5B illustrate the structural support member and the seal plate before and after a weld joint is formed; and
FIG 6 is a flowchart illustrating a method for manufacturing an end effector assembly.

### DETAILED DESCRIPTION

Detailed embodiments of the present disclosure are disclosed herein; however the disclosed embodiments are merely exemplary of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

As mentioned above, conventional electrosurgical forceps include end-effector assemblies that include a pair of opposing jaw members each having an insulating structural support member and a thin seal plate, which may include a knife blade slot adapted to receive a knife blade or portion thereof. Typically, the structural support member and the thin seal plate of the jaw members are stamped or over-molded together.

Structural integrity of the jaw members is provided by their respective structural support members. The insulative support electrically isolates tissue from the thin seal plate and, in some instances, may also be configured to provide additional structural integrity the jaw member. Because the overall structural integrity of the jaw members is provided by their respective structural support members, the seal plates typically are manufactured relatively thin and provide minimal, if any, structural integrity to each respective jaw member.

Conventional seal plates include an electrically conductive tissue contacting surface configured to cause an effect to tissue, e.g., a tissue seal. As mentioned above, in some instances the seal plate may include a slot defined there along configured to receive a knife blade, or portion thereof.

Normally, the slot of the seal plate will be configured to align with a groove or slot located on the structural support member in such a manner that the knife blade, or portion thereof, may translate therein. For the reasons stated above, having end effector assemblies including a structural support member and a seal plate configured in such a manner may prove to be time-consuming and costly to manufacture.

The present disclosure provides an end-effector assembly that includes opposing jaw members each of which includes a structural support member and a seal plate that includes a slot, wherein the seal plates are configured in such a manner that the knife blade, or portion thereof, may translate within the slots of the seal plates independently from their respective structural support members. That is, the seal plates may each have a thickness that allows the knife blade to translate within the slots of the seal plates without extending into their respective structural support members.

Additionally, because of the thickness of the seal plates, the structural support members and their respective seal plates may each be secured to one another via a weld or the like and alignment issues are obviated.

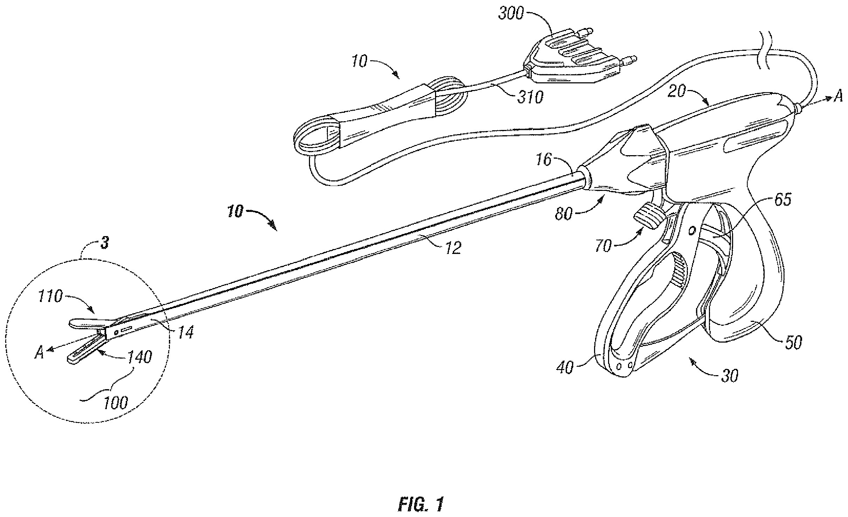

Turning now to FIG. 1, one embodiment of an electrosurgical forceps 10 is shown. For the remainder of the disclosure it will be assumed that the electrosurgical forceps is a bipolar forceps; keeping in mind that any suitable electrosurgical forceps may be employed with the present disclosure. Bipolar forceps 10 is shown for use with various electrosurgical procedures and generally includes a housing 20, a handle assembly 30, a rotating assembly 80, a trigger assembly 70, a drive assembly 130 (not shown), and an end effector assembly 100, which may be operatively connected to the drive assembly 130. End effector assembly 100 includes opposing jaw members 110 and 140, which mutually cooperate to grasp, seal and, in some cases, divide large tubular vessels and large vascular tissues. Although the majority of the figure drawings depict a bipolar forceps 10 for use in connection with endoscopic surgical procedures, the present disclosure may be used for more traditional open surgical procedures. For the purposes herein, the forceps 10 is described in terms of an endoscopic instrument; however, it is contemplated that an open version of the forceps may also include the same or similar operating components and features as described below.

Forceps 10 includes a shaft 12, which has a distal end 14 dimensioned in such a manner that a drive rod 132 of drive assembly 130 mechanically engages the end effector assembly 100 and a proximal end 16 mechanically engages the housing 20. In the drawings and in the descriptions that follow, the term "proximal," as is traditional, will refer to the end of the forceps 10 that is closer to the user, while the term "distal" will refer to the end that is farther from the user.

Handle assembly 30 includes a fixed handle 50 and a movable handle 40. Fixed handle 50 is integrally associated with housing 20 and handle 40 is movable relative to fixed handle 50. Fixed handle 50 may include one or more ergonomic enhancing elements to facilitate handling, e.g., scallops, protuberances, elastomeric material, etc.

Movable handle 40 of handle assembly 30 is ultimately connected to drive assembly 130 including drive rod 132, which together mechanically cooperate to impart movement of jaw members 110 and 140 to move from an open position, wherein the jaw members 110 and 140 are disposed in spaced relation relative to one another, to a clamping or closed position, wherein the jaw members 110 and 140 cooperate to grasp tissue therebetween.

Rotating assembly 80 is operatively associated with the housing 20 and is rotatable approximately 180 degrees about a longitudinal axis "A-A" defined through shaft 12 (see FIG. 1).

Forceps 10 also includes an electrosurgical cable 310 that connects the forceps 10 to a source of electrosurgical energy, e.g., a generator 500 (shown schematically). It is contemplated that generators such as those sold by Valleylab - a division of Tyco Healthcare LP, located in Boulder Colorado may be used as a source of electrosurgical energy, e.g., Ligasure™ Generator, FORCE EZ™ Electrosurgical Generator, FORCE FX™ Electrosurgical Generator, FORCE 1C™, FORCE 2™ Generator, SurgiStat™ II or other suitable generators which may perform different or enhanced functions.

For a more detailed description of handle assembly 30, movable handle 40, rotating assembly 80, electrosurgical cable 310 (including line-feed configurations and/or connections), and drive assembly 130 reference is made to commonly owned Patent Application No., 10/369,894, filed on February 20, 2003, published as US2003229344, entitled VESSEL

### SEALER AND DIVIDER AND METHOD OF MANUFACTURING THE SAME.

End effector assembly 100 includes opposing jaw members 110 and 140. Jaw members 110 and 140 are generally symmetrical and include similar component features that cooperate to effect the sealing and/or dividing of tissue. As a result and unless otherwise noted, only jaw member 110 and the operative features associated therewith are described in detail herein but as can be appreciated, many of these features apply to jaw member 140 as well.

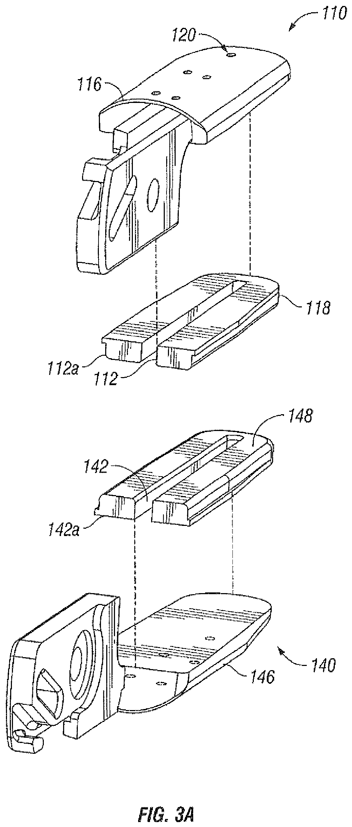

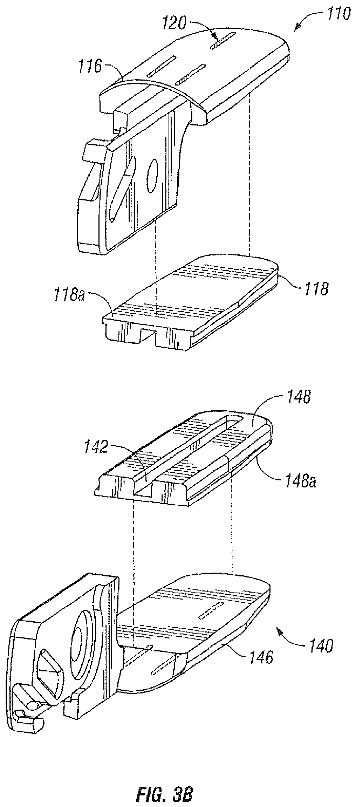

Turning now to FIGS. 3A and 3B, jaw member 110 is shown. Jaw member 110 includes a structural support member 116 and an electrically conductive seal plate 118 (hereinafter seal plate 118) defining a slot 112 therein. The structural support 116 is dimensioned to mechanically engage, as explained in more detail below, the seal plate 118. Similarly, jaw member 140 includes a structural support member 146 and an electrically conducive seal plate 148 defining a slot 142 therein.

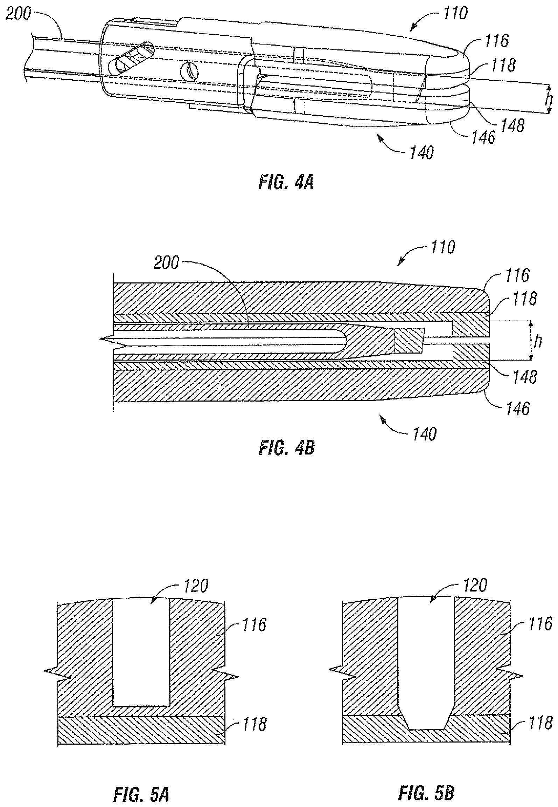

With reference to FIGS. 4A and 4B, seal plates 118 and 148 are configured in such a manner that when jaw members 110 and 140 are in a closed configuration, a knife blade 200, or portion thereof, translates within slot 112 of seal plate 118 and slot 142 of seal plate 148 independent of structural members 116 and 148, respectively. That is, seal plates 118 and 148, or portions thereof, have a combined thickness, when in a closed configuration, that is greater than a height "h" of knife blade 200. For example, each of seal plates 118 and 148 of jaw member 110 and 140, respectively, may each have a thickness that is at least greater than one-half the height "h" of knife blade 200. As a result, knife blade 200 is incapable of or prevented from extending into structural members 116 and 146 and alignment and tolerance issues are altogether avoided during manufacture.

In other words, 118 and 148 of jaw members 110 and 140, respectively, have thicknesses that may not necessarily be equal to one another, but ultimately equal to the height "h" of knife blade 200 when the jaw members are in a closed configuration.

Seal plates 118 and 148 may also have a thickness that varies (i.e., non-uniform) from a proximal end to a distal end, or intermediate therebetween, of each of the seal plates. For example, seal plates 118 and 148 each may have a proximal end that has a thickness that is slightly larger than a thickness at a distal end thereof depending on a particular purpose.

Seal plates 118 and 148 each include a top surface 118a and 148a, respectively, which are herein defined as the surface that secures to the structural support member and a bottom surface, which are herein defined as surface that contacts tissue. Slots 112 and 142 extend distally from a proximal end 112a and 142a of their respective seal plates 118, 148 and provide a path for longitudinal translation of the knife blade 200 therein. Slots 112 and 142 may be opened at the top surface of their respective seal plates (FIG. 3A). Alternatively, slots 118 and 148 may be closed at the top surface of their respective seal plates (FIG. 3B).

Seal plates 118 and 148 defining knife blade slots 112 and 142, respectively, are configured to join to their respective structural members via one or more known manufacturing techniques, e.g., ultrasonic welding, heat welding, arc welding, and so on. Welding techniques suitable for joining structural member 116 to seal plate 118 include but are not limited to resistance welding, gas welding, energy beam welding, explosion welding, and solid state welding.

With reference to FIGS. 3A and 3B, structural support member 116 is shown and is manufactured from any suitable material known in the art including but not limited to metal, plastic and the like. Structure support member 116 is configured to couple to seal plate 118, such that jaw members 110 and 140 are moveable from an open configuration to a closed configuration wherein they may cooperate to effect the sealing and/or dividing of tissue.

Located on structural support members 116 and 140, or portions thereof, are one or more bored apertures 120 (FIGS. 3A and 3B). Apertures 120 may have any suitable shape, such as holes, slots, grooves, and so on, which are configured to extend within structural support 116 and are dimensioned for joining seal plate 118, or portion thereof, to structural support member 116, as shown in FIGS. 5A or 5B. For example, aperture 120 may be configured such that structural support member 116 and seal plate 118 may be joined together via a weld formed therebetween. With this purpose in mind, aperture 120 does not fully extend to seal plate 118 (FIG. 5A). As a result, a portion of structural support 116 is kept between aperture 120 and the top surface 118a of seal plate 118. The portion kept between structural support 116 and seal plate 118 forms part of the weld (FIG. 5B).

Structural members 116 and 146 may be joined to their respective seal plates 118, 148 via one or more of the above welding techniques or by other fastening methods known in the art, e.g., soldering or brazing.

In use, prior to sealing tissue, jaw members 110 and 140 may be initially in an open configuration, each disposed at an angle θ relative to the longitudinal axis "X. When tissue is ready to be grasped for sealing, a user positions tissue between jaw members 110 and 140, and squeezes handle 40 which, in turn, causes jaw members 110 and 140 to move from the open configuration to a closed configuration. After tissue is grasped between jaw members 110 and 140, electrosurgical energy may be transmitted to the jaw members 110 and 140 effecting a tissue seal therebetween (e.g., coagulation).

If tissue is to be cut, while tissue is grasped between jaw members 110 and 140, a user activates knife blade 200 such that knife blade 200 extends longitudinally through slots 112 and 142 of seal plates 118 and 148, respectively, effecting a tissue cut therebetween.

Upon completion of treating target tissue (e.g., sealing and/or cutting), a user releases handle 40, which, in turn, causes jaw members 110 and 140 to return to their initial open configuration, wherein they will again be disposed at an angle θ relative to the longitudinal axis "X".

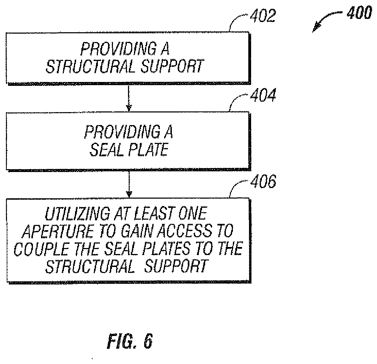

With reference to FIG. 6, a method 400 for manufacturing an end effector assembly for use with a bipolar forceps is shown. At step 402, a structural support including at least one aperture associated with a top surface thereof is provided. At step 404, a seal plate defining a slot configured to translate a knife blade therethrough is provided. And at step 406, one or more apertures configured to gain access to couple the seal plates to the structural support is utilizied.

From the foregoing and with reference to the various figure drawings, those skilled in the art will appreciate that certain modifications can also be made to the present disclosure without departing from the scope of the same.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. An end effector assembly (100) for use with a forceps, comprising:
a pair of opposing jaw members (110, 140) operatively connected to each other about a pivot, each jaw member including a structural support member (116, 146) having an electrically conductive seal plate (118, 148) secured thereto, each of the seal plates defining a slot (112, 142) having a depth and configured to translate a knife blade therethrough, the combined depth of the slots being greater than the height of the knife blade, wherein each of the structural support members includes at least one aperture (120) extending therein and associated with a top surface thereof, the at least one aperture configured to facilitate structural engagement of the structural support member to a respective seal plate, wherein the seal plates each include a top surface (118a, 148a), which is defined as the surface that secures to the structural support member, and a bottom surface, which is defined as the surface that contacts tissue.

2. The end effector assembly according to claim 1, wherein the seal plates are secured to their respective structural support members via at least one of welding, soldering and brazing.

3. The end effector assembly according to claim 2, wherein the type of weld is selected from resistance welding, ultrasonic welding, gas welding, energy beam welding, explosion welding, and solid state welding.

4. The end effector assembly according to any one of the preceding claims, wherein the slots extend through the full thickness of the seal plates.

5. The end effector assembly according to any one of the preceding claims, wherein the seal plates vary in thickness.

6. A method for manufacturing an end effector assembly (100) for use with a forceps, the end effector assembly comprising a pair of opposing jaw members (110, 140) operatively connected to each other about a pivot, each jaw member including a structural support member (116, 146) having an electrically conductive seal plate (118, 148) secured thereto, the method comprising for each jaw member the steps of:
providing a structural support (116, 146), the structural support including at least one aperture (120) associated with a top surface thereof;
providing an electrically conductive seal plate (118, 148), the seal plate defining a slot (112, 142) configured to translate a knife blade therethrough; and
utilizing the at least one aperture to gain access to couple the seal plate to the structural support;
wherein the combined depth of the slots is greater than a height of the knife blade, wherein the seal plates each include a top surface (118a, 148a), which is defined as the surface that secures to the structural support member, and a bottom surface, which is defined as the surface that contacts tissue.

7. The method of according to claim 6, wherein the step of utilizing comprises coupling the seal plate to the structural support by welding, soldering or brazing.

8. The method according to claim 7, wherein the welding is resistance welding, ultrasonic welding, gas welding, energy beam welding, explosion welding or solid state welding.

9. The end effector assembly of any one of claims 1 to 5 or the method of any one of claims 6 to 8, wherein the slots are closed at the top surface of their respective seal plates.

10. The end effector assembly of any one of claims 1 to 5 or 9 or the method of any ones of claims 6 to 8 or 9, wherein the aperture is configured such that the structural support member and the seal plate are joined together via a weld formed therebetween.

11. The end effector assembly of claim 10 or the method of claim 10, wherein a portion of the structural support member formed by the aperture not fully extending to the seal plate is kept between the aperture and the top surface of the seal plate, and wherein the portion forms part of the weld.

## Patentansprüche

1. Endeffektoranordnung (100) zur Verwendung mit einer Zange, umfassend:
ein Paar von gegenüberliegenden Klemmbackenelementen (110, 140), die um einen Drehpunkt herum betriebsmäßig miteinander verbunden sind, wobei jedes Klemmbackenelement ein strukturelles Stützelement (116, 146) mit einer daran gesicherten elektrisch leitenden Dichtungsplatte (118, 148) aufweist, wobei jede der Dichtungsplatten einen Schlitz (112, 142) definiert, der eine Tiefe hat und konfiguriert ist, um eine Messerklinge dort hindurch zu verschieben, wobei die kombinierte Tiefe der Schlitze größer als die Höhe der Messerklinge ist, wobei jedes der strukturellen Stützelemente zumindest eine Öffnung (120) aufweist, die sich darin erstreckt und mit einer oberen Fläche davon verbunden ist, wobei die zumindest eine Öffnung konfiguriert ist, um einen strukturellen Eingriff des strukturellen Stützelements mit einer entsprechenden Dichtungsplatte zu ermöglichen, wobei die Dichtungsplatten jeweils eine obere Fläche (118a, 148a), die als die Fläche definiert ist, die sich an dem strukturellen Stützelement sichert, und eine untere Fläche, die als die Fläche definiert ist, die Gewebe kontaktiert, aufweisen.

2. Endeffektoranordnung nach Anspruch 1, wobei die Dichtungsplatten über zumindest eines von Schweißen, Löten und Hartlöten an ihren entsprechenden strukturellen Stützelementen gesichert sind.

3. Endeffektoranordnung nach Anspruch 2, wobei die Schweißart aus Widerstandsschweißen, Ultraschallschweißen, Gasschweißen, Energiestrahlschweißen, Sprengschweißen und Festkörperschweißen ausgewählt ist.

4. Endeffektoranordnung nach einem der vorstehenden Ansprüche, wobei sich die Schlitze durch die ganze Dicke der Dichtungsplatten erstrecken.

5. Endeffektoranordnung nach einem der vorstehenden Ansprüche, wobei die Dichtungsplatten in der Dicke variieren.

6. Verfahren zur Herstellung einer Endeffektoranordnung (100) zur Verwendung mit einer Zange, wobei die Endeffektoranordnung ein Paar von gegenüberliegenden Klemmbackenelementen (110, 140) umfasst, die um einen Drehpunkt herum betriebsmäßig miteinander verbunden sind, wobei jedes Klemmbackenelement ein strukturelles Stützelement (116, 146) mit einer daran gesicherten elektrisch leitenden Dichtungsplatte (118, 148) aufweist, wobei das Verfahren für jedes Klemmbackenelement die folgenden Schritte umfasst:
Bereitstellen einer strukturellen Stütze (116, 146), wobei die strukturelle Stütze zumindest eine Öffnung (120) aufweist, die mit einer oberen Fläche davon verbunden ist;
Bereitstellen einer elektrisch leitenden Dichtungsplatte (118, 148), wobei die Dichtungsplatte einen Schlitz (112, 142) definiert, der konfiguriert ist, um eine Messerklinge dort hindurch zu verschieben; und
Verwenden der zumindest einen Öffnung, um Zugang zu erhalten, um die Dichtungsplatte mit der strukturellen Stütze zu koppeln;
wobei die kombinierte Tiefe der Schlitze größer als eine Höhe der Messerklinge ist, wobei jede der Dichtungsplatten jeweils eine obere Fläche (118a, 148a), die als die Fläche definiert ist, die sich an dem strukturellen Stützelement sichert, und eine untere Fläche, die als die Fläche definiert ist, die Gewebe kontaktiert, aufweisen.

7. Verfahren nach Anspruch 6, wobei der Schritt von einem Verwenden ein Koppeln der Dichtungsplatte mit der strukturellen Stütze durch Schweißen, Löten oder Hartlöten umfasst.

8. Verfahren nach Anspruch 7, wobei das Schweißen Widerstandsschweißen, Ultraschallschweißen, Gasschweißen, Energiestrahlschweißen, Sprengschweißen oder Festkörperschweißen ist.

9. Endeffektoranordnung nach einem der Ansprüche 1 bis 5 oder Verfahren nach einem der Ansprüche 6 bis 8, wobei die Schlitze an der oberen Fläche ihrer entsprechenden Dichtungsplatten geschlossen sind.

10. Endeffektoranordnung nach einem der Ansprüche 1 bis 5 oder 9 oder Verfahren nach einem der Ansprüche 6 bis 8 oder 9, wobei die Öffnung konfiguriert ist, so dass das strukturelle Stützelement und die Dichtungsplatte über eine dazwischen ausgebildete Schweißnaht miteinander verbunden sind.

11. Endeffektoranordnung nach Anspruch 10 oder Verfahren nach Anspruch 10, wobei ein Abschnitt des strukturellen Stützelements, der durch die Öffnung gebildet ist, die sich nicht ganz bis zu der Dichtungsplatte erstreckt, zwischen der Öffnung und der oberen Fläche der Dichtungsplatte gehalten wird und wobei der Abschnitt einen Teil der Schweißnaht bildet.

## Revendications

1. Ensemble effecteur terminal (100) pour utilisation avec un forceps, comprenant :
une paire d'éléments de mors opposés (110, 140) raccordés en service l'un à l'autre autour d'un pivot, chaque élément de mors comprenant un élément de support structurel (116, 416) ayant une plaque d'étanchéité électroconductrice (118, 148) qui lui est fixée, chacune des plaques d'étanchéité définissant une fente (112, 142) ayant une profondeur et configurée pour y déplacer une lame de couteau, la profondeur combinée des fentes étant supérieure à la hauteur de la lame de couteau, dans lequel chacun des éléments de support structurels comprend au moins une ouverture (120) qui s'y étend et associée à sa surface supérieure, la au moins une ouverture étant configurée pour faciliter un engagement structurel de l'élément de support structurel avec une plaque d'étanchéité respective, dans lequel les plaques d'étanchéité comprennent chacune une surface supérieure (118a, 148a), qui est définie comme la surface qui se fixe à l'élément de support structurel, et une surface inférieure, qui est définie comme la surface qui vient en contact avec un tissu.

2. Ensemble effecteur terminal selon la revendication 1, dans lequel les plaques d'étanchéité sont fixées à leurs éléments de support structurels respectifs via au moins une soudure, une brasure tendre et une forte brasure.

3. Ensemble effecteur terminal selon la revendication 2, dans lequel le type de soudure est choisi parmi une soudure par résistance, une soudure ultrasonique, une soudure autogène, une soudure par faisceau énergétique, une soudure par explosion et une soudure à l'état solide.

4. Ensemble effecteur terminal selon l'une quelconque des revendications précédentes, dans lequel les fentes s'étendent sur toute l'épaisseur des plaques d'étanchéité.

5. Ensemble effecteur terminal selon l'une quelconque des revendications précédentes, dans lequel les plaques d'étanchéité varient en épaisseur.

6. Procédé de fabrication d'un ensemble effecteur terminal (100) pour utilisation avec un forceps, l'ensemble effecteur terminal comprenant une paire d'éléments de mors opposés (110, 140) raccordés en service l'un à l'autre autour d'un pivot, chaque élément de mors comprenant un élément de support structurel (116, 146) ayant une plaque d'étanchéité électroconductrice (118, 148) qui lui est fixée, le procédé comprenant pour chaque élément de mors les étapes consistant à :
fournir un support structurel (116, 146), le support structurel comprenant au moins une ouverture (120) associée à sa surface supérieure ;
fournir une plaque d'étanchéité électroconductrice (118, 148), la plaque d'étanchéité définissant une fente (112, 142) configurée pour y déplacer une lame de couteau ; et
utiliser la au moins une ouverture pour y accéder afin de coupler la plaque d'étanchéité au support structurel ;
dans lequel la profondeur combinée des fentes est supérieure à une hauteur de la lame de couteau, dans lequel les plaques d'étanchéité comprennent chacune une surface supérieure (118a, 148a), qui est définie comme la surface qui se fixe à l'élément de support structurel, et une surface inférieure, qui est définie comme la surface qui vient en contact avec un tissu.

7. Procédé selon la revendication 6, dans lequel l'étape d'utilisation comprend le couplage de la plaque d'étanchéité au support structurel par soudure, brasure tendre ou forte brasure.

8. Procédé selon la revendication 7, dans lequel la soudure est une soudure par résistance, une soudure ultrasonique, une soudure autogène, une soudure par faisceau énergétique, une soudure par explosion ou une soudure à l'état solide.

9. Ensemble effecteur terminal selon l'une quelconque des revendications 1 à 5 ou procédé selon l'une quelconque des revendications 6 à 8, dans lequel les fentes sont fermées à la surface supérieure de leurs plaques d'étanchéité respectives.

10. Ensemble effecteur terminal selon l'une quelconque des revendications 1 à 5 ou 9 ou procédé selon l'une quelconque des revendications 6 à 8 ou 9, dans lequel l'ouverture est configurée de sorte que l'élément de support structurel et la plaque d'étanchéité soient assemblés conjointement via une soudure formée entre eux.

11. Ensemble d'effecteur terminal selon la revendication 10 ou procédé selon la revendication 10, dans lequel une partie de l'élément de support structurel formé par l'ouverture qui ne s'étend pas complètement jusqu'à la plaque d'étanchéité est maintenue entre l'ouverture et la surface supérieure de la plaque d'étanchéité, et dans lequel la partie fait partie intégrante de la soudure.
